(19) Europäisches Patentamt / European Patent Office / Office européen des brevets

(11) **EP 4 092 107 A1**

(12) **EUROPEAN PATENT APPLICATION**
published in accordance with Art. 153(4) EPC

(43) Date of publication:
**23.11.2022 Bulletin 2022/47**

(21) Application number: **21741077.8**

(22) Date of filing: **15.01.2021**

(51) International Patent Classification (IPC):
*C12N 5/071* (2010.01)        *C12N 5/0735* (2010.01)
*C12N 5/077* (2010.01)        *C12N 5/10* (2006.01)
*C12Q 1/06* (2006.01)

(52) Cooperative Patent Classification (CPC):
**C12N 5/06; C12N 5/10; C12Q 1/06**

(86) International application number:
**PCT/JP2021/001152**

(87) International publication number:
**WO 2021/145402 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.01.2020  JP 2020004997**

(71) Applicant: FUJIFILM Corporation
**Tokyo 106-8620 (JP)**

(72) Inventors:
• **MURAKAMI Yuta**
  **Ashigarakami-gun, Kanagawa 258-8577 (JP)**

• **NAGASE Masaya**
  **Ashigarakami-gun, Kanagawa 258-8577 (JP)**
• **MAEDA Kiyohiro**
  **Ashigarakami-gun, Kanagawa 258-8577 (JP)**
• **SHIRAISHI Yasushi**
  **Ashigarakami-gun, Kanagawa 258-8577 (JP)**
• **KOCHI Masahiro**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **METHOD FOR PRODUCING PLURIPOTENT STEM CELLS CAPABLE OF DIFFERENTIATING INTO SPECIFIC CELLS, AND APPLICATION THEREOF**

(57)    An object of the present invention is to provide a method of producing a pluripotent stem cell capable of differentiating into a specific cell. According to the present invention, there is provided a method for producing a pluripotent stem cell capable of differentiating into a specific cell, the method including (i) a step of measuring, in a pluripotent stem cell as a sample, a phenotype associated with induction of epithelial-mesenchymal transition before differentiation induction; and (ii) a step of acquiring a pluripotent stem cell capable of differentiating into a specific cell using the measured phenotype as an indicator. According to the present invention, there are further provided a method of selecting a pluripotent stem cell capable of differentiating into a specific cell; a pluripotent stem cell capable of differentiating into a specific cell; a production method of a differentiated cell; a differentiated cell; and a method for quality evaluation of a pluripotent stem cell.

**Description**

Technical Field

The present invention relates to a method of producing a pluripotent stem cell capable of differentiating into a specific cell and a method of selecting a pluripotent stem cell capable of differentiating into a specific cell. The present invention relates to a pluripotent stem cell capable of differentiating into a specific cell, where the pluripotent stem cell is obtained by the above method. The present invention relates to a method of producing a differentiated cell. The present invention relates to a differentiated cell obtained by the above method of producing a differentiated cell. Further, the present invention also relates to a method for quality evaluation of a pluripotent stem cell.

Background Art

In the field of regenerative medicine, research for inducing differentiation of a pluripotent stem cell into a specific cell is underway to obtain various differentiated cells.

The pluripotent stem cell is defined as a cell that has both the ability (the differentiation pluripotency) to differentiate into all cells that constitute a living body and the self-replication ability. However, in reality, it is known that the differentiation potency is not constant between donors from which pluripotent stem cells are derived or between established clones and that there are cells having high differentiation potency and cells having low differentiation potency.

Patent Document 1 discloses a method of determining the differentiation potency into a human myocardial cell, which is characterized by including a step of analyzing the expression of N-CADHERIN in a cell population containing human stem cells. Patent Document 2 discloses a method of selecting a high-quality induced pluripotent stem cell (iPS cell) by measuring the DNA methylation of a specific gene such as SNAII.

Prior Art Documents

Patent Documents

    Patent Document 1: JP2010-158206A
    Patent Document 2: JP2019-106999A

**SUMMARY OF THE INVENTION**

Pluripotent stem cells are said to be cells having differentiation pluripotency with which they can differentiate into all kinds of cells that constitute the living body. However, in fact, it is known that the differentiation potency is not constant among donors and clones. A differentiated cell is obtained by stepwise differentiating a cell by culturing for a relatively long period of time using complicated culture conditions, and thus, in order to efficiently produce a differentiated cell product, it is required to select a pluripotent stem cell having high differentiation potency before differentiation induction and use it as a cell material. However, according to the method disclosed in JP2010-158206A, a differentiated cell cannot be selected at the stage of iPS cells, and it can be selected only after differentiation into the myocardial progenitor cell. Further, in the method disclosed in JP2019-106999A, DNA methylation is fixed after the establishment of iPS cells, and thus the property changes that has occurred in the process of culturing cannot be observed.

Another object to be achieved by the present invention is to provide a method of producing a pluripotent stem cell capable of differentiating into a specific cell and a method of selecting a pluripotent stem cell capable of differentiating into a specific cell. An object to be achieved by the present invention is to provide a pluripotent stem cell capable of differentiating into a specific cell, where the pluripotent stem cell is obtained by the above method. Another object to be achieved by the present invention is to provide a method of producing a differentiated cell. Another object to be achieved by the present invention is to provide a differentiated cell obtained by the above method of producing a differentiated cell. Further, another object to be achieved by the present invention is to provide a method for quality evaluation of a pluripotent stem cell.

To achieve the above-described objects, the inventors of the present invention carried out diligent studies and measured phenotypes of twelve iPS cell clones associated with the induction of epithelial-mesenchymal transition before the differentiation induction, and as a result of the measurement, it was possible to classify the phenotypes thereof into eight clones not having the above-described phenotype and the four clones having the above-described phenotype. As a result of inducing the differentiation of these cells into myocardial cells and ectodermal cells, it was found that all the four clones having the above-described phenotype have poor differentiation efficiency. From the above results, the inventors of the present invention have found that in the pluripotent stem cells, it is possible to sort a pluripotent stem cell capable of differentiating into a specific cell by using, as an indicator, the phenotype associated with the induction of epithelial-mesenchymal transition before the differentiation induction. The present invention has been completed based on the above findings.

That is, according to the present invention, the following inventions are provided.

[1] A method for producing a pluripotent stem cell capable of differentiating into a specific cell, the method comprising:

(i) a step of measuring, in a pluripotent stem cell as a sample, a phenotype associated with induction of epithelial-mesenchymal transition before differentiation induction; and
(ii) a step of acquiring a pluripotent stem cell capable of differentiating into a specific cell using the measured phenotype as an indicator.

[2] A method of selecting a pluripotent stem cell capable of differentiating into a specific cell, from a human pluripotent stem cell population, the method comprising:

(i) a step of measuring, in a pluripotent stem cell as a sample, a phenotype associated with induction of epithelial-mesenchymal transition before differentiation induction; and
(ii) a step of selecting a pluripotent stem cell capable of differentiating into a specific cell using the measured phenotype as an indicator.

[3] The method according to [1] or [2], in which the phenotype is an expression level of a gene associated with the epithelial-mesenchymal transition.
[4] The method according to [3], in which the gene associated with the epithelial-mesenchymal transition is an N-cadherin gene or a Vimentin gene.
[5] The method according to [1] or [2], in which the phenotype is a consumption amount or a secretion amount of a metabolite associated with the epithelial-mesenchymal transition.
[6] The method according to [5], in which the metabolite is a metabolite belonging to a glutamic acid synthesis pathway or glycolysis.
[7] The method according to [5] or [6], in which the metabolite is L-glutamic acid or lactic acid.
[8] The method according to [1] or [2], in which the phenotype is a change in cell morphology associated with the epithelial-mesenchymal transition.
[9] The method according to [8], in which the change in cell morphology is a change in nuclear area or a change in a ratio of nuclear area/cytoplasmic area.
[10] The method according to any one of [1] to [9], in which the specific cell is a differentiated cell derived from ectoderm or mesoderm.
[11] The method according to any one of [1] to [10], in which the specific cell is a myocardial cell.
[12] A pluripotent stem cell capable of differentiating into a specific cell, which is obtained by the method according to any one of [1] to [11].
[13] A producing method for a differentiated cell, comprising:

a step of obtaining a pluripotent stem cell capable of differentiating into a specific cell, by the method according to any one of [1] to [11]; and
a step of inducing differentiation of the pluripotent stem cell capable of differentiating into a specific cell, obtained in the above step.

[14] The method according to [13], in which the differentiated cell is a cell derived from mesoderm or ectoderm.
[15] A differentiated cell which is obtained by the method according to [13] or [14].
[16] a method for quality evaluation of a pluripotent stem cell, the method comprising:

(i) a step of measuring, in a pluripotent stem cell as a sample, a phenotype associated with induction of epithelial-mesenchymal transition before differentiation induction; and
(ii) a step of evaluating an ability to differentiate into a specific cell using the measured phenotype as an indicator.

According to the present invention, it is possible to produce and select a pluripotent stem cell capable of differentiating into a specific cell. According to the present invention, it is possible to provide a pluripotent stem cell capable of differentiating into a specific cell. According to the present invention, it is possible to provide a method of producing a differentiated cell, and a differentiated cell. According to the present invention, there is provided a method for quality evaluation of a pluripotent stem cell.

## BRIEF DESCRIPTION OF THE DRAWINGS

Fig. 1 shows results of an analysis of cTnT-positive cells by a flow cytometer.

Fig. 2 is a graph in which an expression level of NANOG is compared between a high-producing clone group and a low-producing clone group.

Fig. 3 is a graph in which an expression level of N-CADHERIN is compared between the high-producing clone group and the low-producing clone group. * indicates P-value < 0.01.

Fig. 4 is a graph in which an expression level of VIMENTIN is compared between the high-producing clone group and the low-producing clone group. * indicates P-value < 0.01.

Fig. 5 is a graph in which a nuclear area is compared between the high-producing clone group and the low-producing clone group. * indicates P-value < 0.01.

Fig. 6 is a graph in which a ratio of N/C is compared between the high-producing clone group and the low-producing clone group. * indicates P-value < 0.01.

Fig. 7 is a graph in which a secretion amount of glutamic acid, which is a metabolite, is compared between the high-producing clone group and the low-producing clone group. * indicates P-value < 0.01.

Fig. 8 is a graph in which a secretion amount of lactic acid, which is a metabolite, is compared between the high-producing clone group and the low-producing clone group. * indicates P-value < 0.01.

Fig. 9 is a graph showing an expression level of N-CADHERIN before the differentiation induction into ectoderm.

Fig. 10 is a graph showing an expression level of VIMENTIN before the differentiation induction into ectoderm.

Fig. 11 is a graph showing an expression level of PAX6 after the differentiation induction into ectoderm.

## DESCRIPTION OF THE PREFERRED EMBODIMENTS

Hereinafter, embodiments for carrying out the present invention will be described in detail.

The abbreviations in the present specification have the following meanings.

bFGF: basic Fibroblast Growth Factor
BMP4: Bone Morphogenetic Protein 4
cTnT: cardiac Troponin T
DMEM: Dulbecco's Modified Eagle Medium
DMEM/F12 (DMEM Ham's F-12): Dulbecco's Modified Eagle Medium/Nutrition Mixture F-12 Ham
D-PBS: Dulbecco's Phosphate Buffered Saline
EDTA: Ethylenediaminetetraacetic acid
Erk: Extracellular Signal-regulated Kinase
ESG: Embryonal stem cell-specific gene
FGF: Fibroblast growth factor
GAPDH: glyceraldehyde-3-phosphate dehydrogenase
GSK: Glycogen Synthesis Kinase
Klf: Kruppel-like factor
LC/MS/MS: Liquid Chromatography/Mass Spectrometry/Mass Spectrometry
LGR5: Leucine-rich repeat-connecting G-protein coupled receptor 5
LIF: Leukemia inhibitory factor
Oct: octamer-binding transcription factor
PAX6: paired box 6
PCR: polymerase chain reaction
ROCK: Rho-associated coiled-coil forming kinase
RT-PCR: Reverse Transcription polymerase chain reaction
SCF: Stem cell factor
Sox: sex determining region Y (SRY)-box
SANAI1 (Snail Family Transcriptional Repressor 1)
Stat3: Signal Transducer and Activator of Transition 3)
VEGF: Vascular Endothelial Growth Factor

[1] Method of producing pluripotent stem cell, selection method for pluripotent stem cell, and pluripotent stem cell

The present invention relates to a method of producing a pluripotent stem cell capable of differentiating into a specific cell and includes the following steps:

(i) a step of measuring, in a pluripotent stem cell as a sample, a phenotype associated with induction of epithelial-mesenchymal transition before differentiation induction; and
(ii) a step of acquiring a pluripotent stem cell capable of differentiating into a specific cell using the measured phenotype as an indicator.

The present invention is a method of selecting a pluripotent stem cell capable of differentiating into a specific cell, from a human pluripotent stem cell population, the method comprising:

(i) a step of measuring, in a pluripotent stem cell as a sample, a phenotype associated with induction of epithelial-mesenchymal transition before differentiation induction; and
(ii) a step of selecting a pluripotent stem cell capable of differentiating into a specific cell using the measured phenotype as an indicator.

According to the present invention, it is possible to select a pluripotent stem cell capable of differentiating into a specific cell by using, as an indicator, the phenotype associated with the induction of epithelial-mesenchymal transition before the differentiation induction. In a case where a cell that is not capable of differentiating into a specific cell or a cell that is weakly capable of differentiating into a specific cell is removed, the production cost can be reduced, and the production can be stabilized.

The "pluripotent stem cell" refers to a cell having the ability (the differentiation pluripotency) to differentiate into all cells that constitute a living body and the ability (the self-replication ability) to generate daughter cells having the same differentiation potency as the mother pluripotent stem cell through cell division. The differentiation pluripotency can be evaluated by transplanting an evaluation target cell into a nude mouse and testing for the presence or absence of formation of teratoma that includes cells of the respective three germ layers (ectoderm, mesoderm, and endoderm). The self-replication ability can be evaluated from the fact that, in a case of being subcultured, cells are proliferated and the characteristics of the proliferated cells are not changed by the subculture.

Examples of the pluripotent stem cell include an embryonic stem cell (an ES cell), an embryonic germ cell (an EG cell), and an induced pluripotent stem cell (an iPS cell); however, examples thereof are not limited thereto as long as a cell has both differentiation pluripotency and self-replication ability. It is preferable to use an ES cell or an iPS cell, and it is more preferable to use an iPS cell. The pluripotent stem cell is preferably a cell of a mammal (for example, a primate such as a human or a chimpanzee or a rodent such as a mouse or a rat), and more preferably a human cell. In the most preferred aspect of the present invention, a human iPS cell is used as the pluripotent stem cell.

The ES cell can be established, for example, by culturing an early embryo before implantation, an inner cell mass constituting the above early embryo, or a single blastomere (Manipulating the Mouse Embryo, A Laboratory Manual, Second Edition, Cold Spring Harbor Laboratory Press (1994); Thomason, J. A. et al., Science, 282, 1145-1147 (1998)). As the early embryo, an early embryo prepared by nuclear transfer of a somatic cell nucleus may be used (Wilmut et al. (Nature, 385, 810 (1997)), Cibelli et al. (Science, 280, 1256) (1998)), Akira Iriya et al. (Protein, nucleic acid, and enzyme, 44, 892 (1999)), Baguisi et al. (Nature Biotechnology, 17, 456 (1999)), Wakayama et al. (Nature, 394, 369 (1998)); Nature Genetics, 22, 127 (1999); Proc. Natl. Acad. Sci. USA, 96, 14984 (1999)), Rideout III et al. (Nature Genetics, 24, 109 (2000), Tachibana et al. (Human Embryonic Stem Cells Delivered by Somatic Cell Nuclear Transfer, Cell (2013) in press). As the early embryo, a parthenogenetic embryo may be used (Kim et al. (Science, 315, 482-486 (2007)), Nakajima et al. (Stem Cells, 25, 983-985 (2007)), Kim. et al. (Cell Stem Cell, 1,346-352 (2007)), Revazova et al. (Cloning Stem Cells, 9, 432-449 (2007)), Revazova et al. (Cloning Stem Cells, 10, 11-24 (2008)). In addition to the above-described papers, regarding the preparation of an ES cell, the following can be referenced, Strelchenko N. et al. Reprod Biomed Online. 9: 623-629, 2004; Klimanskaya I., et al. Nature 444: 481-485, 2006; Chung Y., et al. Cell Stem Cell 2: 113-117,2008; Zhang X., et al. Stem Cells 24: 2669-2676, 2006; Wassarman, P. M. et al. Methods in Energy, Vol. 365, 2003. It is noted that a fused ES cell obtained by cell fusion of an ES cell with a somatic cell is also included in the embryonic stem cell that is used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

The EG cell can be established by, for example, culturing a primordial germ cell in the presence of LIF, bFGF, and SCF (Matsui et al., Cell, 70, 841-847 (1992), Shamblott et al., Proc. Natl. Acad. Sci. USA, 95 (23), 13726-13731 (1998), Turnpenny et al., Stem Cells, 21 (5), 598-609, (2003)).

The "induced pluripotent stem cell (the iPS cell)" is a cell that has pluripotency (multiple differentiation potency) and proliferation ability and that is produced by reprogramming a somatic cell by introducing reprogramming factors or the like. The induced pluripotent stem cell exhibits properties similar to the ES cell. The somatic cell that is used for preparing an iPS cell is not particularly limited and may be a differentiated somatic cell or an undifferentiated stem cell. In addition, the origin thereof is not particularly limited; however, it is preferable to use a somatic cell of mammals (for example, primates such as a human and a chimpanzee, rodents such as a mouse and a rat), it is more preferable to use a human somatic cell. The iPS cell can be produced by various methods reported so far. In addition, it is naturally expected that an iPS cell producing method to be developed in the future will be applied.

The most basic method of preparing an iPS cell is a method of introducing four transcription factors, Oct3/4, Sox2, Klf4, and c-Myc, into a cell using a virus (Takahashi K, Yamanaka S: Cell 126 (4), 663-676, 2006; Takahashi, K, et al: Cell 131 (5), 861-72, 2007). It has been reported that human iPS cells have been established by introducing four factors, Oct4, Sox2, Lin28, and Nanog (Yu J, et al.: Science 318 (5858), 1917-1920, 2007). It has also been reported that iPS cells are established by introducing three factors excluding c-Myc (Nakagawa M, et al: Nat. Biotechnol. 26 (1), 101-106, 2008), two factors of Oct3/4 and Klf4 (Kim J B, et al: Nature 454 (7204), 646-650, 2008), or only Oct3/4 (Kim J B, et al: Cell 136 (3), 411-419, 2009). In addition, a method of introducing a protein, which is an expression product of a gene, into a cell (Zhou H, Wu S, Joo JY, et al: Cell Stem Cell 4, 381-384,2009; Kim D, Kim CH, Moon JI, et al.: Cell Stem Cell 4, 472-476, 2009) has also been reported. On the other hand, it has been also reported that it is possible to improve the preparation efficiency or reduce the factors to be introduced, by using an inhibitor BIX-01294 for a histone methyltransferase G9a, a histone deacetylase inhibitor valproic acid (VPA), Bay K8644, or the like (Huangfu D, et al: Nat. Biotechnol. 26 (7), 795-797, 2008; Huangfu D, et al: Nat. Biotechnol. 26 (11), 1269-1275, 2008; Silva J, et al: PLoS. Biol. 6 (10), e253, 2008). In addition, gene introducing methods have been studied as well, and techniques using, in addition to retroviruses, the following substances have been developed; lentiviruses (Yu J, et al: Science 318 (5858), 1917-1920, 2007), adenoviruses (Stadtfeld M, et al: Science 322 (5903), 945-949, 2008), plasmids (Okita K, et al: Science 322 (5903), 949-953, 2008), transposon vectors (Woltjen K, Michael IP, Mohseni P, et al: Nature 458, 766-770, 2009; Kaji K, Norrby K, Pac a A, et al: Nature 458, 771-775, 2009; Yusa K, Rad R, Takeda J, et al: Nat Methods 6,363-369, 2009), or episomal vectors (Yu J, Hu K, Smuga-Otto K, Tian S, et al: Science 324, 797-801, 2009).

A cell transformed to the iPS cell, that is, a cell that has undergone initialization (reprogramming) can be selected using, as an indicator, the expression of pluripotent stem cell markers (undifferentiation markers) such as Nanog, Oct4, Fgf-4, Esg-1, and Cript, or the like. The selected cell is collected as the iPS cell.

The iPS cells can be provided from, for example, National University Corporation Kyoto University, or Independent Administrative Institution RIKEN BioResource Center.

In the present specification, the specific cell is any differentiated cell derived from endoderm, mesoderm, or ectoderm.

The differentiated cell derived from the endoderm is not particularly limited; however, examples thereof include a digestive system cell (a liver cell, a bile duct cell, a pancreatic endocrine cell, an acinar cell, a duct cell, an absorptive cell, a goblet cell, a Paneth cell, an intestinal endocrine cell, an intestinal epithelial cell-like cell, or the like) and a cell of a tissue such as lung or thyroid.

The differentiated cell derived from the mesoderm is not particularly limited; however, examples thereof include a hematopoietic cell and a lymphoid cell (a hematopoietic stem cell, an erythrocyte, a platelet, a macrophage, a granulocyte, a helper T cell, a killer T cell, a B lymphocyte, or the like), a vascular system cell (a vascular endothelial cell or the like) a myocardial cell (for example, an atrial cardiac muscle cell, or a ventricular cardiac muscle cell), an osteoblast, a bone cell, a cartilage cell, a tendon cell, an adipocyte, a skeletal muscle cell, and a smooth muscle cell.

The differentiated cell derived from the ectoderm is not particularly limited; however, examples thereof include a nervous system cell (a nerve cell, a glial cell, or the like), a sensory organ cell (crystalline lens, retina, inner ear, or the like), a cutaneous epidermal cell, and a hair follicle.

The specific cell in the present invention is preferably a differentiated cell derived from ectoderm or mesoderm. The specific cell in the present invention is more preferably a differentiated cell derived from mesoderm and particularly preferably a myocardial cell.

The step (i) in the method embodiment of the present invention is a step of measuring, in a pluripotent stem cell as a sample, a phenotype associated with induction of epithelial-mesenchymal transition before differentiation induction.

The "pluripotent stem cell as a sample" may include a cell population capable of differentiating into the specific cell and a cell population not capable of differentiating into the specific cell, may include only cells capable of differentiating into the specific cell, or may contain only cells not capable of differentiating into the specific cell. In the description of the "pluripotent stem cell as a sample", "as a sample" means that the sample is capable of being a material for producing a pluripotent stem cell capable of differentiating into the specific cell, and from the "pluripotent stem cell as a sample", a pluripotent stem cell capable of differentiating into the specific cell is acquired by the step (ii) described later.

The phenotype associated with the induction of epithelial-mesenchymal transition is not particularly limited;

however, examples thereof include the expression level of genes associated with the epithelial-mesenchymal transition, the consumption amount or secretion amount of metabolites associated with the epithelial-mesenchymal transition, and the change in cell morphology associated with the epithelial-mesenchymal transition.

<Expression of gene associated with epithelial-mesenchymal transition>

Epithelial-mesenchymal transition (EMT) is a process through which an epithelial cell loses its cell polarity or cell adhesion function to surrounding cells and changes into a mesenchymal-like cell by gaining migration ability and invasion ability. The epithelial-mesenchymal transition plays an important role in the developmental processes such as mesoderm formation and neural tube formation, and it is conceived to occur in wound healing, tissue fibrosis, and cancer infiltration and metastasis as well. In epithelial cells, E-CADHERIN and the like are highly expressed, whereas in mesenchymal cells, N-CADHERIN, VIMENTIN, and the like are highly expressed. The epithelial-mesenchymal transition is also an important process in embryonic development, and it is conceived that the epithelial-mesenchymal transition is induced in the process of cell differentiation and development, whereby cells acquire migration ability, and the development of various organs progresses. In vitro as well, pluripotent stem cells are epithelial cells expressing E-CADHERIN, and they form epithelial cell-like colonies on a dish.

Examples of the genes associated with the epithelial-mesenchymal transition in the present invention include, which are not limited to, an N-cadherin gene, a Vimentin gene, a Snail gene, a Slug gene, a Fibronectin gene, an ETS1 gene, an $\alpha$-SMA gene, and a Twist gene.

The expression of a gene can be usually analyzed by the production amount of a transcript corresponding to the gene, or the production amount, the activity, and the like of a translation product thereof. The measurement of the expression can be carried out by measuring an mRNA which is a transcript of a gene or a protein which is a translation product of a gene; however, it is preferably carried out by measuring an mRNA or a cDNA which is a reverse transcript thereof.

Table 1 shows the NCBI accession number of each gene.

[Table 1]

| Gene name | NCBI accession Number | |
| --- | --- | --- |
| | Human | Mouse |
| N-cadherin | NM_001308176.2 | NM_07664.5 |
| Vimentin | NM_003380.5 | NM_011701.4 |

Based on the above NCBI accession number, the sequence information of each gene can be acquired, and the expression level of the gene can be measured.

The method of measuring the expression of genes associated with the epithelial-mesenchymal transition is not particularly limited; however, the measurement can be carried out by, for example, the quantitative RT-PCR. RT-PCR is a method of synthesizing cDNA using the measurement target mRNA as a template and amplifying it by PCR using this cDNA as a template. Examples of the quantitative RT-PCR include a method (a real-time PCR) in which PCR is carried out using a primer to which a quencher fluorescent dye and a reporter fluorescent dye are bound to quantify the amount of an amplification product in each cycle, and the amount of a template DNA in a sample is measured from the number of cycles at which the detected fluorescence intensity increases sharply. The quantitative RT-PCR technique is well known in the technical field of the present invention and can also be carried out using a commercially available kit. According to the quantitative RT-PCR, the expression level or the number of copies of a gene can be measured as a relative value with respect to the expression level or the number of copies of a control housekeeping gene (for example, a GAPDH gene). The mRNA of a gene can also be measured by subjecting an amplification product obtained by amplifying the mRNA by the ordinary RT-PCR or the like to gel electrophoresis, staining the gel, and then measuring the band intensity. Alternatively, a DNA chip can be used to detect or quantify the mRNA or cDNA of a gene. The expression level of genes associated with the epithelial-mesenchymal transition can also be measured using a next-generation sequencer. A measurement target cell can be obtained by the partial extraction of cells in the culture step.

As the numerical value indicating the expression level, for example, a cycle threshold (Ct) value can be used in a case where the expression level is measured by the real-time PCR. The Ct value is the number of cycles at which a PCR amplification product reaches a certain amount. In a case where the number of cycles of amplification is plotted on the horizontal axis and the amount of a PCR product is plotted on the vertical axis to create an amplification curve, and a threshold is set for the value of the PCR product, the number of cycles at the point where the threshold and the amplification curve intersect is the Ct value. In a case of measuring the expression level using a fluorescently labeled probe, fluorescence intensity can also be used.

<Secretion and consumption of metabolite associated with epithelial-mesenchymal transition>

The metabolites associated with the epithelial-mesenchymal transition are not particularly limited, and examples thereof include metabolites belonging to the glutamic acid synthesis pathway or glycolysis.

Examples of metabolites belonging to the glutamic acid synthesis pathway include L-glutamic acid, L-glutamine, $\alpha$-ketoglutaric acid, and oxaloacetate.

Examples of the metabolites belonging to glycolysis include glucose, glucose-6-phosphate, fructose-6-phosphate, fructose-1,6-bisphosphate, dihydroxyacetone phosphate, glyceraldehyde-3-phosphate, 1,3-bisphosphoglyceric acid, 3-phosphoglyceric acid, 2-phosphoglyceric acid, phosphoenol pyruvate, pyruvic acid, and lactic acid.

The secretion of a metabolite can be evaluated by measuring the amount of metabolite secreted into the medium of cells. The consumption of a metabolite can be evaluated by measuring the amount of the metabolite consumed in the medium of cells.

The measuring method for the amount of the metabolite secreted in the medium of cells is not particularly limited, and it suffices that the amount of the metabolite in the medium is measured by LC/MS/MS or the like. Alternatively, the measurement can be carried out using a bioanalyzer equipped with a biosensor having an enzyme membrane.

Examples of the change in cell morphology associated with the epithelial-mesenchymal transition include, which are not limited to, a change in the nuclear area and a change in a ratio of nuclear area/cytoplasmic area.

The nuclear area and the ratio of nuclear area/cytoplasmic area can be evaluated by cell morphology analysis by microscopic observation or the like. Specifically, it suffices that a staining image of the nucleus and the cytoplasm is obtained by fluorescent staining using Hoechst or Calcein, and the area values and the ratio of the nucleus and cytoplasm are calculated by image processing. In addition, it is also possible to adopt a method of calculating areas of the nucleus and the cytoplasm from an unstained cell image by utilizing image deep learning or the like.

In the method of producing a pluripotent stem cell and the method of selecting a pluripotent stem cell according to the present invention, first, a pluripotent stem cell can be subjected to expansion culture. The "pluripotent stem cell as a sample" is preferably subjected to the expansion culture in a suitable medium on a plate coated with a feeder cell or a plate coated with a scaffold such as Matrigel (registered trade name). The feeder cell is not particularly limited; however, examples thereof include a mouse embryonic fibroblast (a MEF cell) and a mouse embryonic fibroblast (an STO cell). In the expansion culture, it is preferable that the undifferentiative property of a cell is maintained.

As the medium for the expansion culture, a commercially available medium such as mTeSR (registered trade name) 1 (STEMCELL Technologies Inc.) or StemFlex (registered trade name) can be used. Alternatively or additionally, for example, DMEM, a mixed medium of DMEM and F12 (DMEM/F12 = 1:1), and Knockout™ D-MEM (Thermo Fisher Scientific, Inc.) can be mentioned as the basal medium, and a medium prepared by adding, to any one of the above basal media, any combination of components such as alternative serum (KSR; Knockout™ Serum Replacement (Thermo Fisher Scientific, Inc.)), fetal bovine serum (FBS), non-essential amino acid (NEAA), L-glutamine, 2-mercaptoethanol, and an antibiotic (for example, streptomycin, penicillin, puromycin, mitomycin, or gentamicin), and bFGF can be mentioned as a medium for the expansion culture.

The culture conditions for the expansion culture are preferably conditions of 37°C, 5% $CO_2$, and 20% $O_2$, or the like; however, they are not particularly limited.

By the expansion culture, cells can be cultured until the cell concentration in the culture solution changes from $1 \times 10^4$ cells/ml to $1 \times 10^{10}$ cells/ml.

Subculture may be carried out during the expansion culture. For example, in a case where cells become confluent or subconfluent, a part of the cells can be collected and transferred to another culture vessel, and the culture can be continued. It is preferable to set a cell density low in order to promote differentiation. For example, cells may be plated at a cell density of about $1 \times 10^4$ cells/cm$^2$ to $1 \times 10^6$ cells/cm$^2$.

The pluripotent stem cells as a sample are cells obtained by clonally culturing and proliferating a pluripotent stem cell by the expansion culture. The clonal culture means a culture for obtaining a population of cells, which is formed through the division and the proliferation of one cell by culture, and the cell population obtained by the clonal culture is called a clonal population or simply a clone. The cells obtained by being subjected to clonal culture and proliferation may be regarded to be identical to the original cells before clonal culture and proliferation. In addition, the pluripotent stem cells as a sample may be those obtained from 72 hours before the start of differentiation induction to the time of the start of differentiation induction, may be those obtained from 48 hours before the start of differentiation induction to the time of the start of differentiation induction or may be those obtained from 24 hours before the start of differentiation induction to the time of the start of differentiation induction.

The step (ii) in the method of producing a pluripotent stem cell and the method of selecting a pluripotent stem cell according to the present invention is a step of acquiring or selecting a pluripotent stem cell capable of differentiating into the specific cell by using the measured phenotype as an indicator.

In a case where the phenotype is the expression level of the gene associated with the epithelial-mesenchymal transition, the expression level of the genes associated with the epithelial-mesenchymal transition can be measured in the step (i), where the expression level thereof is normalized based on the expression level of the GAPDH gene. In a case of selecting a pluripotent stem cell of which the normalized expression level measured as described above is lower than a predetermined value, it is possible to acquire a pluripotent stem cell capable of differentiating into the specific cell. In addition, a pluripotent stem cell capable of differentiating into the specific cell may be acquired by excluding pluripotent stem cells of which the normalized expression level is higher than the predetermined value.

In a case where the gene associated with the epithelial-mesenchymal transition is the N-CADHERIN gene, the expression level (the expression level normalized based on the expression level of the GAPDH gene) of the N-CADHERIN gene in the selected pluripotent stem cell is preferably 0.03 times or less and more preferably 0.02 times or less. In addition, in a case where the gene associated with the epithelial-mesenchymal transition is the N-CADHERIN gene, the expression level (the expression level normalized based on the expression level of the GAPDH gene) of the N-CADHERIN gene in the excluded pluripotent stem cells is preferably more than 0.03 times.

In a case where the gene associated with the epithelial-mesenchymal transition is the VIMENTIN gene, the expression level (the expression level normalized based on the expression level of the GAPDH gene) of the VIMENTIN gene in the selected pluripotent stem cells is preferably 0.2 times or less, more preferably 0.15 times or less, and still more preferably 0.1 times or less. In addition, in a case where the gene associated with the epithelial-mesenchymal transition is the VIMENTIN gene, the expression level (the expression level normalized based on the expression level of the GAPDH gene) of the VIMENTIN gene in the excluded pluripotent stem cells is preferably more than 0.2 times.

In a case where the phenotype is the consumption amount or secretion amount of a metabolite associated with the epithelial-mesenchymal transition, the secretion amount of metabolite per cell per 24 hours is measured in the step (i), for example, by a method described in Example 5 described later. In a case where the metabolite is L-glutamic acid or lactic acid, it is possible to acquire a pluripotent stem cell capable of differentiating into the specific cell by selecting a pluripotent stem cell in which the secretion amount of L-glutamic acid or the secretion amount of lactic acid is lower than a predetermined value. In addition, in a case where the metabolite is L-glutamic acid or lactic acid, a pluripotent stem cell capable of differentiating into the specific cell may be acquired by removing pluripotent stem cells in which the secretion amount of L-glutamic acid or the secretion amount of lactic acid is higher than the predetermined value.

In a case where the phenotype is a change in cell morphology associated with the epithelial-mesenchymal transition, for example, the nuclear area or the ratio of nuclear area/cytoplasmic area is measured in the step (i). In a case where the change in cell morphology is a change in the nuclear area, it is possible to acquire a pluripotent stem cell capable of differentiating into the specific cell selecting a pluripotent stem cell of which the nuclear area is lower than a predetermined value. In addition, in a case where the change in cell morphology is a change in the nuclear area, a pluripotent stem cell capable of differentiating into the specific cell may be acquired by removing pluripotent stem cells of which the nuclear area is higher than the predetermined value. In a case where the change in cell morphology is a change in the ratio of nuclear area/cytoplasmic area, it is possible to acquire a pluripotent stem cell capable of differentiating into the specific cell by selecting a pluripotent stem cell of which the ratio of nuclear area/cytoplasmic area is higher than a predetermined value. In addition, in a case where the change in cell morphology is a change in the ratio of nuclear area/cytoplasmic area, a pluripotent stem cell capable of differentiating into the specific cell may be acquired by removing pluripotent stem cells of which the nuclear area is higher than the predetermined value.

In a case where the change in cell morphology is a change in the nuclear area, a pluripotent stem cell in which the nuclear area is small (reduced) can be selected. It is preferable to select a pluripotent stem cell in which the nuclear area (number of pixels) is 700 or less, and it is more preferable to adopt 600 or less as an indicator.

In a case where the change in cell morphology is a change in the ratio of nuclear area/cytoplasmic area, it can be adopted as an indicator that the ratio of nuclear area/cytoplasmic area is high (increased). It is preferable to select a pluripotent stem cell in which the ratio of nuclear area/cytoplasmic area is 0.7 or more, and it is more preferable to select a pluripotent stem cell in which it is 0.75 or more.

A person skilled in the art can appropriately set a reference value for selecting a pluripotent stem cell having differentiation potency into the specific cell based on a value of the above-described phenotype in the pluripotent stem cell known to have differentiation potency into the specific cell.

For example, in two or more clones selected as the "pluripotent stem cell known to have differentiation potency into the specific cell", the average value of the values of the phenotype is set as a reference value, and by the comparison with this reference value, it is also possible to select a pluripotent stem cell capable of differentiating into the specific cell.

The "pluripotent stem cell known to have high differentiation potency into the specific cell" means a pluripotent stem cell that has been revealed that the differentiation potency into the specific cell is relatively high, by the reported publication such as literature or the experiment carried out in advance for evaluating the differentiation potency into the specific cell.

Examples of the "pluripotent stem cell known to have high differentiation potency into the specific cell" include a 01434 clone (McNamara et al., 2018, Cell Systems 7, 359-370) of Fujifilm Cellular Dynamics, Inc.

The "pluripotent stem cell known to have high differentiation potency into the specific cell" can be selected, for example, by evaluating the production rate of a differentiated cell or a progenitor cell thereof, which is derived from mesoderm, endoderm, or ectoderm, and preferably a differentiated cell or a progenitor cell thereof, which is derived from mesoderm or ectoderm. More specifically, the differentiation potency of the pluripotent stem cell into the specific cell can be evaluated by inducing differentiation into, for example, the myocardial cell, a blood cell, a skeletal muscle cell, a nerve cell, a hepatic parenchymal cell, a pancreatic β cell, an intestinal epithelial cell, or a progenitor cell thereof. This is because there is an established experimental system for inducing differentiation of these differentiated cells or progenitor cells thereof.

In the selection of the pluripotent stem cell having differentiation potency into the specific cell, two or more kinds of phenotypes of the pluripotent stem cells as a sample are measured and scored by applying discriminant analysis, whereby it is possible to determine whether or not the pluripotent stem cells as a sample are pluripotent stem cell having differentiation potency into the specific cell. Examples of the discriminant analysis include Fisher's linear discrimination, Mahalanobis' distance-based discrimination, Euclidean distance-based discrimination, multigroup discrimination, variable selection discrimination, and canonical discrimination. These discriminant analyzes can be carried out using various statistical analysis software.

The present invention also includes a pluripotent stem cell capable of differentiating into the specific cell, which is obtained by the production method described above. In a case where the pluripotent stem cell capable of differentiating into the specific cell, which is obtained by the above production method, is induced to differentiate, it is possible to efficiently produce a differentiated cell.

Further, another aspect of the present invention relates to a method for quality evaluation of a pluripotent stem cell, the method including:

(i) a step of measuring, in a pluripotent stem cell as a sample, a phenotype associated with induction of epithelial-mesenchymal transition before differentiation induction; and
(ii) a step of evaluating an ability to differentiate into a specific cell using the measured phenotype as an indicator.

The quality evaluation means predicting whether or not the pluripotent stem cell as a sample has high differentiation potency into the specific cell, before the differentiation induction. Using the phenotype associated with the epithelial-mesenchymal transition induction before the differentiation induction as an indicator, it is possible to evaluate that a pluripotent stem cell as a sample, which is predicted to have high differentiation potency into the specific cell, is an excellent clone and that a pluripotent stem cell as a sample, which is predicted not to have high differentiation potency into the specific cell, is a defective clone.

According to the method for quality evaluation according to the embodiment of the present invention, the production cost can be reduced and the production can be stabilized by excluding the defective clones by the quality evaluation. In addition, in a case where a part of cells are sampled at a plurality of time points in the clonal culture process to monitor the progress of the phenotype associated with the epithelial-mesenchymal transition induction, the deterioration of cell quality in the clonal culture process can be detected, which can also be fed back to the production process. In each of the steps of establishing, expanding, and differentiating an iPS cell, the phenotype associated with the epithelial-mesenchymal transition induction can be measured and monitored as a novel quality reference of the iPS cell.

The step (i) can be carried out in the same manner as the step (i) described in the [1] Production method. The step (ii) can be carried out in the same manner as the step (ii) described in the [1] Production method. In the step (ii), in a case where a phenotype associated with the induction of epithelial-mesenchymal transition before the differentiation induction, the phenotype being measured in the step (i), is compared with the phenotype in a pluripotent stem cell, which is already known to have high differentiation potency into the specific cells, it is possible to evaluate the ability to differentiate into the specific cell. It is preferable that the pluripotent stem cell evaluated to be capable of differentiating into the specific cell is a differentiation induction target. This makes it possible to efficiently produce a differentiated cell product.

An example of the pluripotent stem cell having differentiation potency into the specific cell includes a pluripotent stem cell having differentiation potency into the myocardial cell. Examples of the pluripotent stem cell having differentiation potency into the myocardial cell include a pluripotent stem cell preferably having a myocardial cell production rate of 50% or more, and include a pluripotent stem cell more preferably having a myocardial cell production rate of 60% or more, 70% or more, 80% or more, 90% or more, 100% or more, 110% or more, 120% or more, or 130% or more. In addition, the pluripotent stem cell may be a pluripotent stem cell having a myocardial cell production rate of 140% or more, 150% or more, 160% or more, 170% or more, 180% or more, 190% or more, 200% or more, or 300% or more.

The myocardial cell production rate (%) means (the total number of myocardial cells after differentiation induction/the number of pluripotent stem cells at the start of differentiation induction) $\times$ 100.

As will be described later, the differentiation induction into the myocardial cell can be carried out as follows; cells are cultured in mTeSR (registered trade name) 1, to which a ROCK inhibitor and bFGF are added, on the 0th day, cultured in a $\times$ 0.5 mTeSR (registered trade name) 1 and $\times$ 0.5 DMEM low-glucose medium containing a ROCK inhibitor, bFGF, Activin A, and fetal bovine serum, on the 1st day, cultured in a DMEM low-glucose medium containing bFGF, Activin A, BMP4, and fetal bovine serum on the 2nd day to 7th day, cultured in the same medium containing a Wnt inhibitor and fetal bovine serum on the 8th day, and cultured in the same medium without a Wnt inhibitor on the 9th day to 13th day.

The myocardial cell can be identified by detecting a cell expressing cTnT which is a myocardial cell marker, for example, using an anti-cTnT antibody labeled with a fluorescent dye. The number of myocardial cells can be calculated by measuring the proportion (the cTnT-positive cell rate) of cells expressing cTnT by flow cytometry method using an anti-cTnT antibody or the like through the labeling with a fluorescent dye. The cTnT-positive cell rate can be measured by the flow cytometry method according to the method described in Example 2.

[2] Production method differentiated cell, and differentiated cell

The present invention relates to a method of producing a differentiated cell and includes the following steps;

a step of obtaining a pluripotent stem cell capable of differentiating into a specific cell by the method of producing a pluripotent stem cell capable of differentiating into a specific cell, and
a step of inducing differentiation of the pluripotent stem cell capable of differentiating into a specific cell, obtained in the above step.

In a case where the pluripotent stem cell capable of differentiating into the specific cell, which is obtained by the method of producing a pluripotent stem cell capable of differentiating into the specific cell according to the embodiment of the present invention, is induced to differentiate, it is possible to efficiently produce a differentiated cell.

A step of obtaining a pluripotent stem cell capable of differentiating into a specific cell by the method of producing a pluripotent stem cell capable of differentiating into a specific cell can be carried out based on the description of the [1] Production method.

The "inducing differentiation" refers to acting to differentiate along a specific cell lineage. The method of inducing differentiation into a pluripotent stem cell capable of differentiating into a specific cell is not particularly limited. For example, a commercially available STEMdiff (registered trade name) Trillineage Differentiation Kit (STEMCELL Technologies Inc.) can be used to induce differentiation into each of the endoderm, mesoderm, and ectoderm.

In the differentiation induction into an endodermal cell, for example, a cell can be induced to differentiate into the endodermal cell by using a STEMdiff (registered trade name) Trilineage Endoderm Medium (STEMCELL Technologies Inc.) according to the procedure manual.

In the differentiation induction into a mesodermal cell, for example, a cell can be induced to differentiate into the mesodermal cell by using a STEMdiff (registered trade name) Trilineage Mesoderm Medium (STEMCELL Technologies Inc.) according to the procedure manual.

The differentiation induction into the ectodermal cell can be carried out, for example, by culturing cells under the conditions described in Example 6 described later. Specifically, a cell can be induced to differentiate into the ectodermal cell by using a STEMdiff (registered trade name) Trilineage Ectoderm Medium (STEMCELL Technologies Inc.) according to the procedure manual.

The confirmation of differentiation induction into the endodermal cell, the mesodermal cell, and the ectodermal cell can be confirmed by the expression of each germ layer-specific gene. The method of measuring the expression of each germ layer-specific gene is not particularly limited; however, the measurement can be carried out by, for example, a quantitative RT-PCR method.

The endoderm-specific gene is not particularly limited; however, examples thereof include SOX17 and FOXA2. The mesoderm-specific gene is not particularly limited; however, examples thereof include T and PDGFRA. The ectoderm-specific gene is not particularly limited; however, examples thereof include PAX6 and MAP2.

The differentiation induction into the myocardial cell can be carried out, for example, by culturing cells under the conditions described in Example 1 described later. Specifically, the differentiation induction into the myocardial cell can be carried out as follows; cells are cultured in mTeSR (registered trade name) 1, to which a ROCK inhibitor and bFGF are added, on the 1st day, cultured in a × 0.5 mTeSR (registered trade name) 1 and × 0.5 DMEM low-glucose medium containing a ROCK inhibitor, bFGF, Activin A, and fetal bovine serum, on the 2nd day, cultured in a DMEM low-glucose medium containing bFGF, Activin A, BMP4, and fetal bovine serum on the 3rd day to 8th day, cultured in the same medium containing a Wnt inhibitor and fetal bovine serum on the 9th day, and cultured in the same medium without a Wnt inhibitor on the 10th day to 14th day. As the ROCK inhibitor, for example, H1152, Y27634, and the like can be used. As the Wnt inhibitor, for example, XAV939 can be used. Alternatively, it is also possible to induce differentiation into the myocardial cell using a PSC Cardiomyocyte Difference Kit (Thermo Fisher Scientific, Inc.) according to the procedure manual. The differentiation induction into the myocardial cell can be confirmed by measuring the expression of the myocardial cell marker cTnT by flow cytometry.

In addition, the differentiation induction into the blood cell can be carried out by culturing cells under the conditions described in PCT/JP2019/003336A. Specifically, the differentiation induction into the blood cell can be carried out as follows; cells are cultured in a medium containing BMP4 and Y27634 (a ROCK inhibitor) on the 1st day, bFGF and BMP4 are added on the 2nd day, after confirming the formation of spheroid-like colonies on the 3rd day, the cells are cultured in a medium containing SB431542 (a TGF-β receptor inhibitor), CHIR99021 (a GSK3 inhibitor), bFGF, and BMP4 (on the 3rd day and 4th day), cultured in a medium containing VEGF and bFGF on the 5th day and 6th day, and cultured in a medium containing VEGF, bFGF, IL-6, IGF-1, IL-11, and SCF on the 7th day to 10th day. The differentiation induction into the blood cell can be confirmed by analyzing the expression of CD34 and KDR, which are markers of the blood cell, with a flow cytometer.

The differentiation induction into the neural stem cell can be carried out, for example, by culturing cells under the conditions described in PCT/JP2019/003336A. Specifically, cells can be induced to differentiate into the neural stem cell by using a PSC Natural Induction Medium (Thermo Fisher Scientific, Inc.) according to the procedure manual. The differentiation induction into the neural stem cell can be confirmed, for example, by immunostaining the SOX1 protein, which is a marker of the neural stem cell.

The differentiation induction into the intestinal stem cell-like cell can be carried out, for example, by culturing cells under the conditions described in PCT/JP2019/004553A. Specifically, cells can be cultured in DMEM/F12 containing FGF2 or a GSK-3β inhibitor to induce differentiation into the intestinal stem cell-like cell. The differentiation induction into the intestinal stem cell-like cell can be confirmed, for example, by immunostaining the LGR5 protein, which is a marker of the intestinal stem cell.

The differentiation induction into the intestinal epithelial cell-like cell can be carried out, for example, by culturing cells under the conditions described in PCT/JP2019/004553A. Specifically, cells can be cultured in Advanced DMEM/F-12 containing EGF and forskolin to induce differentiation into the intestinal epithelial cell-like cell. The differentiation induction into the intestinal epithelial cell-like cell can be determined or evaluated using, for example, the expression of an intestinal epithelial cell marker, the incorporation of a peptide, or the induction of expression of a drug metabolizing enzyme via a vitamin D receptor as an indicator.

In a case of being cultured under differentiation conditions to the mesodermal cell other than the conditions described above, according to the method of producing a differentiated cell according to the embodiment of the present invention, the pluripotent stem cell capable of differentiating into the specific cell can be allowed to differentiate into cells derived from mesoderm. The differentiated cell derived from the mesoderm is not particularly limited; however, examples thereof include a hematopoietic cell and a lymphoid cell (a hematopoietic stem cell, an erythrocyte, a platelet, a macrophage, a granulocyte, a helper T cell, a killer T cell, a B lymphocyte, or the like), a vascular system cell (a vascular endothelial cell or the like) a myocardial cell (for example, an atrial cardiac muscle cell, or a ventricular cardiac muscle cell), an osteoblast, a bone cell, a cartilage cell, a tendon cell, an adipocyte, a skeletal muscle cell, and a smooth muscle cell.

In a case of being cultured under differentiation conditions to the ectodermal cell other than the conditions described above, according to the method of producing a differentiated cell according to the embodiment of the present invention, the pluripotent stem cell capable of differentiating into the specific cell can be allowed to differentiate into cells derived from ectoderm. The differentiated cell derived from the ectoderm is not particularly limited; however, examples thereof include a nervous system cell (a nerve cell, a glial cell, or the like), a sensory organ cell (crystalline lens, retina, inner ear, or the like), a cutaneous epidermal cell, and a hair follicle.

In a case of being cultured under differentiation conditions to the endodermal cell, according to the method of producing a differentiated cell according to the embodiment of the present invention, the pluripotent stem cell capable of differentiating into the specific cell can be allowed to differentiate into cells derived from endoderm. The differentiated cell derived from the endoderm is not particularly limited; however, examples thereof include digestive system cell (a liver cell, a bile duct cell, a pancreatic endocrine cell, an acinar cell, a duct cell, an absorptive cell, a goblet cell, a Paneth cell, an intestinal endocrine cell, an intestinal epithelial cell, or the like) and a cell of a tissue such as lung or thyroid.

The present invention also includes a differentiated cell obtained by the above method of producing a differentiated cell. The cell induced to differentiate according to the method of producing a differentiated cell according to the embodiment of the present invention can be used for screening pharmaceutical drug candidate compounds for the treatment of various diseases. For example, in a case where a pharmaceutical drug candidate compound is added, alone or in combination with other drugs, to cells which have been induced to differentiate, it is possible to carry out an evaluation by detecting a change in the morphology or function of the cell, the increase or decrease in various factors, the gene expression profiling, or the like.

A cell induced to differentiate by using the cell induced to differentiate according to the method of producing a differentiated cell according to the embodiment of the present invention can be used to prepare a tissue that can be used in the field of regenerative medicine. A person skilled in the art shall be familiar with the method of transplanting the prepared tissue to a patient.

The present invention will be further specifically described with reference to Examples; however, the present invention is not limited by Examples.


Examples


<Example 1> Differentiation induction into myocardial cell


IPS cell clones A to L were prepared from peripheral blood mononuclear cells having different donor origins according to the method described in JP5984217B. mTeSR (registered trade name) 1 (STEMCELL Technologies Inc.) and Matrigel (registered trade name) (Corning Inc.) were used for culturing iPS cells, and the cells were treated with a 0.5 mmol/L EDTA solution (Thermo Fisher Scientific, Inc.) for 7 minutes for collection and then subcultured. Under the above conditions, the iPS cells were subjected to the expansion culture up to two T225 flasks.

The iPS cells were proliferated under the above conditions until the confluency reached about 80%, the cells were subsequently detached to single cells with TrypLE Select (registered trade name, Thermo Fisher Scientific, Inc.) and collected, and the cell concentration was adjusted to $3.0 \times 10^6$ cells/mL in mTeSR (registered trade name) 1 to which 1 $\mu$mol/L H1152 (FUJIFILM Wako Pure Chemical Corporation), 25 $\mu$g/ml Gentamicin (Thermo Fisher Scientific, Inc.), and 100 ng/ml bFGF (FUJIFILM Wako Pure Chemical Corporation) were added in terms of final concentration. At the time of carrying out the detachment to single cells, a part of the used culture solution was sampled and used in the metabolite analysis described later, and a part of the cell suspension was sampled in the gene expression analysis and metabolite analysis of iPS cells described later. 15 ml of the suspension of the above cells was added to a 30 ml single-use bioreactor (ABLE Corporation) and subjected to spinner culture at a rotation speed of 40 rpm. After 2 to 4 hours from the start of the spinner culture, the cell suspension was adjusted to a final liquid volume of 30 ml in the same medium and continuously subjected to the spinner culture as it was.

1 day after the start of the spinner culture, the medium was replaced with a medium consisting of, in terms of final concentration, 1 $\mu$mol/L H1152, 25 $\mu$g/ml Gentamicin, 100 ng/ml bFGF, 24 ng/mL Activin A, 5% fetal bovine serum (GE Healthcare), $\times$ 0.5 mTeSR (registered trade name) 1, and $\times$ 0.5 DMEM Low-glucose (Thermo Fisher Scientific, Inc.), and then the spinner culture was continued.

Two days after the start of the spinner culture, a part of the culture solution was sampled to measure the number of cells, the cell concentration was adjusted to $1.0 \times 10^6$ cells/ml in a medium consisting of, in terms of final concentration, 25 $\mu$g/ml Gentamicin, 100 ng/ml bFGF, 24 ng/ml Activin A, 40 ng/ml BMP4, 10% fetal bovine serum, and DMEM low-glucose, and then the spinner culture was continued. From 3 days to 7 days after the start of the spinner culture, the medium was changed daily with the same medium, and then the spinner culture was continued.

8 days after the start of the spinner culture, a part of the culture solution was sampled to measure the number of cells, the cell concentration was adjusted to $1.0 \times 10^6$ cells/ml in a medium consisting of, in terms of final concentration, 25 $\mu$g/ml gentamicin, 16.25 $\mu$g/ml XAV939 (Sigma-Aldrich Co., LLC), 10% fetal bovine serum, and DMEM low-glucose, and then the spinner culture was continued. From 9 days to 13 days after the start of the spinner culture, the medium was changed every two days with the same medium excluding XAV939, and then the spinner culture was continued.

14 days after the start of spinner culture, a part of the culture solution was sampled to measure the number of cells. Table 2 shows the number of cells finally obtained after the differentiation induction.

[Table 2]

| Clone name | Total cell number after differentiation induction into myocardial cells ($\times 10^6$ cells) |
|---|---|
| A | 143.5 |
| B | 534.7 |
| C | 4.9 |
| D | 24.8 |
| E | 573.5 |

(continued)

| Clone name | Total cell number after differentiation induction into myocardial cells ($\times 10^6$ cells) |
|---|---|
| F | 212.9 |
| G | 8.4 |
| H | 315.4 |
| I | 173.1 |
| J | 94.6 |
| K | 555.2 |
| L | 46.6 |

<Example 2> Analysis of iPS cell-derived myocardial cell aggregate by flow cytometer

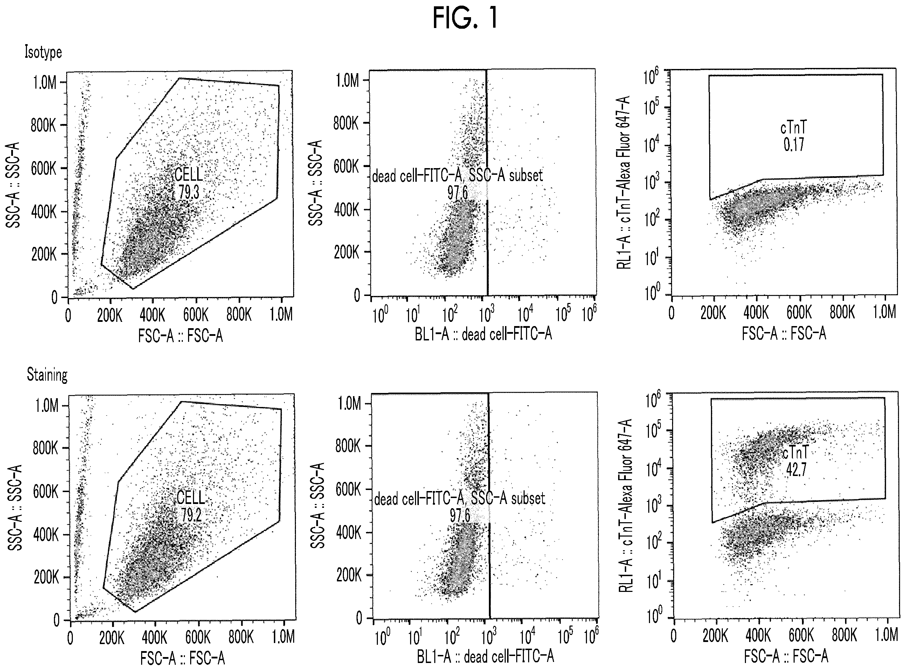

The cell aggregate 14 days after the start of the spinner culture was separated into single cells by TrypLE (registered trade name) Select, and then dead cells were stained with a Live/Dead (registered trade name) Fixable Green Dead Cell Stain Kit. After washing with D-PBS (Thermo Fisher Scientific, Inc.), cells were treated with formaldehyde (Sigma-Aldrich Co., LLC) at a final concentration of 4% to fix the cells. An anti-cardiac Troponin T (cTnT) antibody (Abcam plc, ab8295) was diluted to 1/250 in D-PBS containing 0.1% Saponin (Merck Millipore) and 2% fetal bovine serum in terms of final concentration, added to $0.5 \times 10^6$ cells of the obtained fixed cell, and treated at room temperature for 1 hour. At the same time, as the isotype control, a sample to which IgG1 isotype Control Murine Myeloma (Sigma-Aldrich Co., LLC, M5284) diluted to 1/25 had been added was arranged in parallel. Subsequently, an Alexa Fluor (registered trade name) 647-labeled Goat anti-mouse IgG1 antibody (Thermo Fisher Scientific, Inc., A21240) was diluted to 1/500, added to the cells, and treated at room temperature for 30 minutes. The obtained labeled cells were analyzed using a flow cytometer (Thermo Fisher Scientific, Inc., Attune Nxt). After gating with forward light scattering and lateral light scattering, and then gating live cells, the cTnT-positive cell rate was calculated from the comparison with the isotype sample. Fig. 1 shows examples of results of the flow cytometry and the gating of the obtained clone A, and Table 3 shows the cTnT-positive cell rate of each clone, the total number of myocardial cells calculated from the total number of cells and the cTnT-positive cell rate after differentiation induction, and the myocardial cell production rate (the total number of myocardial cells/the number of iPS cells at the start of differentiation induction) based on the number of cells at the start of differentiation induction (at the start of spinner culture). From the results in Table 3, clones A, B, E, F, H, I, J, and K were defined as a high-producing clone group of the myocardial cell, and clones C, D, G, and L were defined as a low-producing clone group thereof, and then they were used for the following analysis.

[Table 3]

| Clone name | cTnT-positive cell rate (%) | Total cell number of myocardial cells ($\times 10^6$ cells) | Myocardial cell production rate (%) |
|---|---|---|---|
| A | 42.7 | 61.3 | 136.2 |
| B | 14.4 | 77.0 | 171.1 |
| C | 0.1 | 0.0 | 0.0 |
| D | 22.8 | 5.6 | 12.5 |
| E | 25.5 | 146.2 | 325.0 |
| F | 16.8 | 35.8 | 79.5 |
| G | 1.1 | 0.1 | 0.2 |
| H | 44.9 | 141.6 | 314.7 |
| I | 41.6 | 72.0 | 160.0 |
| J | 40.1 | 37.9 | 84.3 |
| K | 11.6 | 64.4 | 143.1 |
| L | 16.9 | 7.9 | 17.5 |

<Example 3> Gene expression analysis of iPS cell

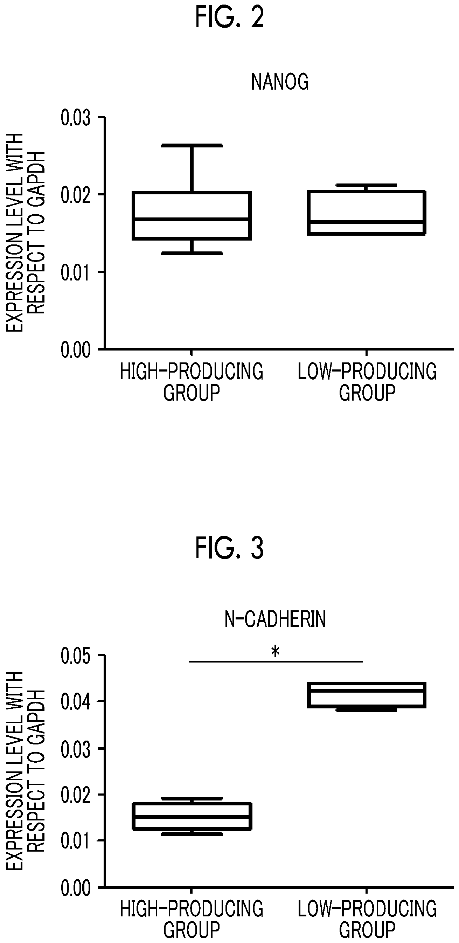

RNA was extracted by using RNeasy Plus Mini Kit (Qiagen) from the iPS cells in Example 1, which had been sampled immediately before the start of differentiation induction. The above RNA was subjected to reverse transcription into cDNA using a High capacity RNA-to-cDNA Kit (Thermo Fisher Scientific, Inc.), and a Taqman (registered trade name) Gene Expression Assay (Thermo Fisher Scientific, Inc.) was used to quantify the expression level of each gene (normalized based on the expression level of GAPDH). The IDs of Taqman (registered trade name) Probes used are shown in Table 4. Table 5 shows the results of the evaluation of expression level of each gene by Taqman Probe (normalized based on the GAPDH expression level). Obtained results are divided into the high-producing clone group and the low-producing clone group to summarize the results as box-and-whisker plots, which are shown in Fig. 2 to Fig. 4.

It was confirmed that the expression level of the undifferentiation marker NANOG is not significantly different between the two groups, whereas the EMT-associated genes (N-cadherin and Vimentin) are expressed significantly low in the high-producing clones (* in the figure indicates P-value < 0.01).

[Table 4]

| Gene name | Taqman Gene Expression Assay ID |
|-----------|--------------------------------|
| NANOG | Hs02387400_g1 |
| N-cadherin | Hs00983056_m1 |
| Vimentin | Hs00958111_m1 |

[Table 5]

| Clone name | Group | NANOG | N-cadherin | Vimentin |
|-----------|-------|-------|-----------|----------|
| A | High -producing group | 0.0146 | 0.0146 | 0.0764 |
| B | | 0.0164 | 0.0114 | 0.0907 |
| E | | 0.0170 | 0.0158 | 0.0507 |
| F | | 0.0170 | 0.0192 | 0.0567 |
| H | | 0.0141 | 0.0175 | 0.0500 |
| I | | 0.0263 | 0.0182 | 0.0761 |
| J | | 0.0214 | 0.0119 | 0.0770 |
| K | | 0.0124 | 0.0147 | 0.0653 |
| C | Low-producing group | 0.0148 | 0.0439 | 0.322 |
| D | | 0.0153 | 0.0408 | 0.415 |
| G | | 0.0176 | 0.0439 | 0.398 |
| L | | 0.0212 | 0.0382 | 0.254 |

From the above, it was shown that iPS cell clones having relatively high myocardial cell productivity commonly have low expression levels of the EMT-associated genes.

<Example 4> Morphology analysis of iPS cell

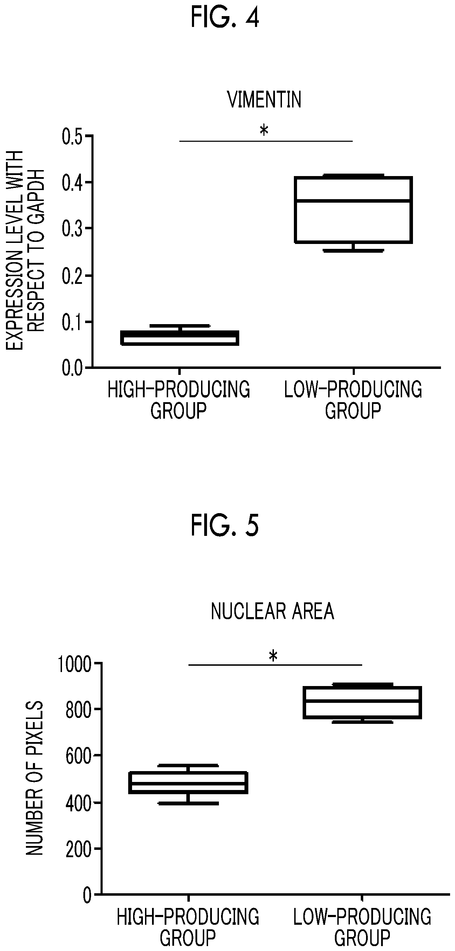

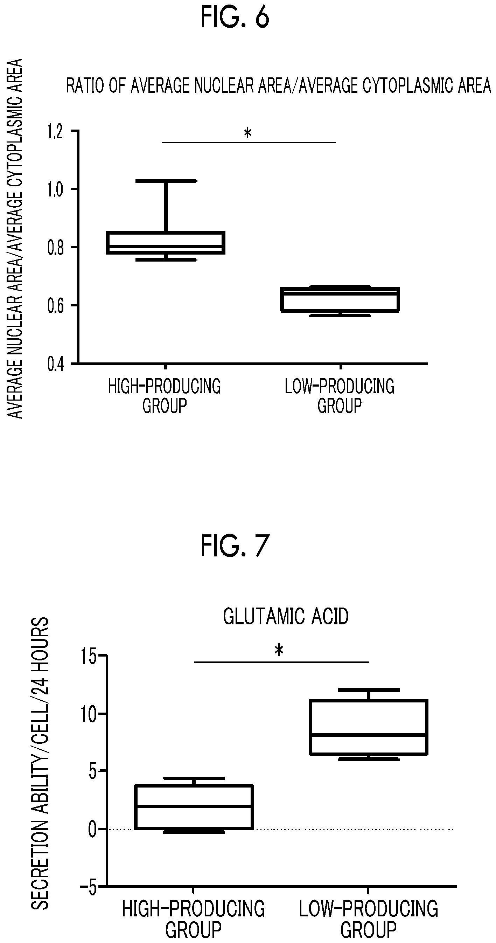

A culture solution to which 100-fold diluted Calcein AM (Dojindo Molecular Technologies. Inc.) and Hoechst (registered trade name) 33342 (Dojindo Molecular Technologies. Inc.) had been added was added to a medium of the iPS cells immediately before the start of induction, and the cells were allowed to stand in an incubator for 30 minutes. Images of the stained cells were captured under a fluorescence microscope, whereby cytoplasmic staining images and nuclear staining images were acquired. The acquired images were analyzed, and the area average of each of the cytoplasm and the nucleus was calculated by using ImageJ, which is an image processing software. The ratio of N/C was calculated as average nuclear area/average cytoplasmic area. The results are shown in Table 6, and Figs. 5 and 6. It has been confirmed that the clone group having high myocardial cell productivity has a small nuclear area and a high ratio of N/C (* in the figures indicates P-value < 0.01). A large nuclear area or a low ratio of N/C is a morphological change that occurs due to cell elongation by EMT induction, and these morphological characteristics indicate that the myocardial cell productivity is relatively reduced by EMT induction.

[Table 6]

| Clone name | Group | Nuclear area | Ratio of N/C |
|---|---|---|---|
| A | High -producing group | 431 | 0.85 |
| B | | 395 | 1.03 |
| E | | 470 | 0.78 |
| F | | 485 | 0.79 |
| H | | 477 | 0.76 |
| I | | 539 | 0.80 |
| J | | 555 | 0.84 |
| K | | 486 | 0.80 |
| C | Low-producing group | 848 | 0.64 |
| D | | 746 | 0.66 |
| G | | 826 | 0.56 |
| L | | 909 | 0.64 |

<Example 5> Metabolite analysis of iPS cell

1 ml each of a fresh medium that had been used at the time of the medium exchange carried out 24 hours before carrying out the detachment to single cells and a post-culture medium that had been sampled at the time of carrying out the detachment to single cells in Example 1 was prepared. After centrifuging the above medium at 300 G for 5 minutes, the supernatant was stored at -80°C. The sample was subjected to a deproteinization treatment with an automatic pretreatment device (C2MAP, manufactured by Shimadzu Corporation), and subjected to a medium wide target analysis (LC/MS/MS Method Package for Cell Culture Profiling, manufactured by Shimadzu Corporation) with LC/MS/MS (a NexeraX2/LCMS-8060 system, manufactured by Shimadzu Corporation), thereby acquiring data on the total 51 components in the supernatant, shown in Table 7. The secretion amount per cell at each point of the culture process was calculated from the peak area value according to the following Calculation Expression (1).

Calculation Expression (1):

secretion amount per cell = (peak area value of post-culture medium - peak area value of fresh medium)/number of cells

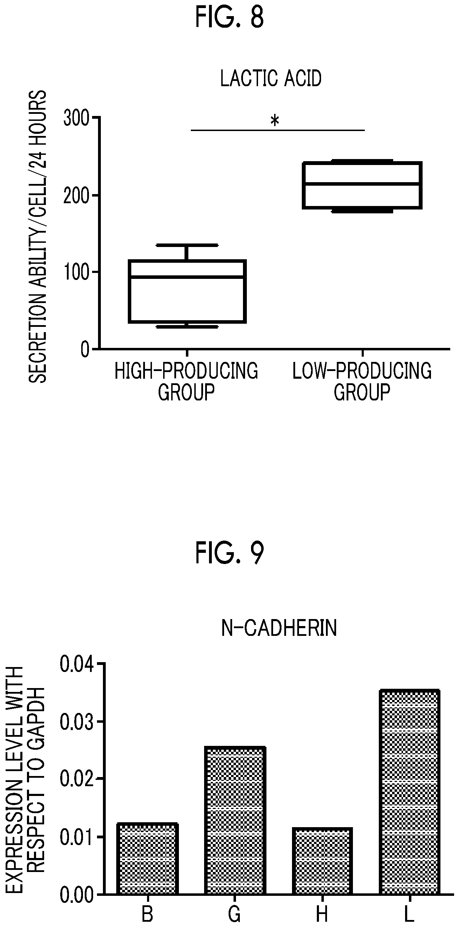

As a result of classifying the results with respect to the high-productivity clone group and the low-productivity clone group and identifying components having a significant difference therebetween, glutamic acid (Fig. 7) and lactic acid (Fig. 8) were extracted (Table 8). The unit of the numerical value shown in Table 8 is Arbitrary Unit (AU)/cell ($\times 10^6$ cells). That is, it has been shown that the secretion amounts of glutamic acid and lactic acid are high in the high-producing clone group. In addition, it has been shown that the low-producing clones have an EMT phenotype since the production amounts of these metabolites have been reported to increase during EMT induction.

[Table 7]

| Amino acid-associated | Asparagine | Sugar | Hexose (glucose) |
|---|---|---|---|
| | Serine | | Threonic acid |
| | Leucine | Vitamin | Pantothenic acid |
| | Histidine | | Pyridoxine |
| | Glutamic acid | | Pyridoxal |
| | Tyrosine | | Choline |
| | Isoleucine | | Riboflavin |
| | Valine | | Nicotinamide |
| | Tryptophan | | Folic acid |
| | Phenylalanine | Nucleic acid-associated | Deoxycytidine |
| | Arginine | | Hypoxanthine |
| | Lysine | | Thymidine |
| | Aspartic acid | | Uracil |
| | Alanine | | Cytidine |
| | Glutamine | | Uric acid |
| | Threonine | Others | Succinic acid |
| | Cysteine | | Lactic acid |
| | Proline | | Pyruvic acid |
| | Glycine | | Malic acid |
| | Methionine | | Glyceric acid |
| | 2-Aminoadipic acid | | |
| | Kynurenine | | |
| | N-acetyl aspartic acid | | |
| | Alanyl glutamine | | |
| | Cystine | | |
| | Methionine sulfoxide | | |
| | Cystathionine | | |
| | Hydroxyproline | | |
| | 4-aminolactic acid | | |
| | Ornithine | | |
| | Citrulline | | |

[Table 8]

| Clone name | Group | Glutamic acid | Lactic acid |
|---|---|---|---|
| A | High -producing group | 0.65 | 30.00 |
| B | | -0.28 | 38.20 |
| E | | 1.89 | 35.18 |
| F | | 2.90 | 76.02 |
| H | | -0.15 | 114.23 |
| I | | 2.14 | 134.00 |
| J | | 4.02 | 110.40 |
| K | | 4.40 | 111.80 |
| C | Low-producing group | 6.02 | 178.67 |
| D | | 7.95 | 244.02 |
| G | | 8.40 | 233.84 |
| L | | 12.02 | 194.22 |

<Example 6> Differentiation induction into ectodermal cell

Among the twelve iPS cell clones, clones B, G, H, and L were selected and subjected to the expansion culture in the same manner as in Example 1. After proliferating iPS cells until the confluency reached about 80%, the cells were detached to single cells with TrypLE Select and collected, and the cell concentration was adjusted to $1 \times 10^6$ cells/ml in a STEMdiff (registered trade name) Trilineage Ectoderm Medium (STEMCELL Technologies Inc.) containing, in terms of final concentration, 10 $\mu$mol/l Y-27632 (FUJIFILM Wako Pure Chemical Corporation) and plated on a 6-well plate coated with Matrigel (registered trade name). From the next day, the medium was changed daily with a STEMdiff (registered trade name) Trilineage Ectoderm Medium and cultured for 7 days after plating.

<Example 7> Correlation analysis between gene expression of iPS cell and ectodermal cell differentiation rate

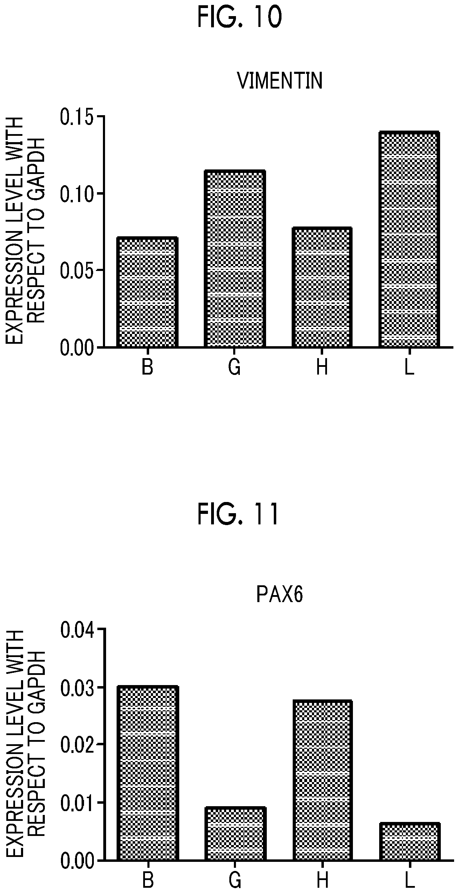

After culturing the cells for 7 days according to the method described in Example 5, RNA was collected using an RNeasy Plus Mini Kit. The cDNA was prepared in the same manner as in Example 4, and the expression level of the PAX6 gene, which is an ectoderm marker gene, was analyzed using a Taqman (registered trade name) Gene Expression Assay (ID: Hs01088114_m1). In addition, the RNA immediately before the start of differentiation induction of each iPS clone was also recovered, and the expression levels of the EMT genes N-cadherin and Vimentin were measured (ID: Hs00983056_m1 and Hs00958111_m1, normalized based on the expression level of GAPDH). The results are shown in Table 9, and Figs. 9, 10, and 11. It has been shown that all the iPS clones (the clones G and L) having low expression of the EMT genes have a relatively high PAX6 expression level after ectoderm induction, that is, have a high ectodermal differentiation efficiency.

[Table 9]

| | Expression level of N-cadherin before differentiation induction | Expression level of Vimentin before differentiation induction | Expression level of PAX6 after differentiation induction into ectoderm |
|---|---|---|---|
| B | 0.0123 | 0.0711 | 0.0300 |
| G | 0.0255 | 0.114 | 0.00907 |
| H | 0.0115 | 0.0770 | 0.0275 |
| L | 0.0353 | 0.139 | 0.00640 |

**Claims**

1. A method for producing a pluripotent stem cell capable of differentiating into a specific cell, the method comprising:

   (i) a step of measuring, in a pluripotent stem cell as a sample, a phenotype associated with induction of epithelial-mesenchymal transition before differentiation induction; and
   (ii) a step of acquiring a pluripotent stem cell capable of differentiating into a specific cell using the measured phenotype as an indicator.

2. A method of selecting a pluripotent stem cell capable of differentiating into a specific cell, from a human pluripotent stem cell population, the method comprising:

   (i) a step of measuring, in a pluripotent stem cell as a sample, a phenotype associated with induction of epithelial-mesenchymal transition before differentiation induction; and
   (ii) a step of selecting a pluripotent stem cell capable of differentiating into a specific cell using the measured phenotype as an indicator.

3. The method according to claim 1 or 2,
   wherein the phenotype is an expression level of a gene associated with the epithelial-mesenchymal transition.

4. The method according to claim 3,
   wherein the gene associated with the epithelial-mesenchymal transition is an N-cadherin gene or a Vimentin gene.

5. The method according to claim 1 or 2,
   wherein the phenotype is a consumption amount or a secretion amount of a metabolite associated with the epithelial-mesenchymal transition.

6. The method according to claim 5,
   wherein the metabolite is a metabolite belonging to a glutamic acid synthesis pathway or glycolysis.

7. The method according to claim 5 or 6,
   wherein the metabolite is L-glutamic acid or lactic acid.

8. The method according to claim 1 or 2,
   wherein the phenotype is a change in cell morphology associated with the epithelial-mesenchymal transition.

9. The method according to claim 8,
   wherein the change in cell morphology is a change in nuclear area or a change in a ratio of nuclear area/cytoplasmic area.

10. The method according to any one of claims 1 to 9,
    wherein the specific cell is a differentiated cell derived from ectoderm or mesoderm.

11. The method according to any one of claims 1 to 10,
    wherein the specific cell is a myocardial cell.

12. A pluripotent stem cell capable of differentiating into a specific cell, which is obtained by the method according to any one of claims 1 to 11.

13. A method of producing a differentiated cell, comprising:

    a step of obtaining a pluripotent stem cell capable of differentiating into a specific cell, by the method according to any one of claims 1 to 11; and
    a step of inducing differentiation of the pluripotent stem cell capable of differentiating into a specific cell, obtained in the above step.

14. The method according to claim 13,
    wherein the differentiated cell is a cell derived from mesoderm or ectoderm.

**15.** A differentiated cell which is obtained by the method according to claim 13 or 14.

**16.** A method for quality evaluation of a pluripotent stem cell, the method comprising:

(i) a step of measuring, in a pluripotent stem cell as a sample, a phenotype associated with induction of epithelial-mesenchymal transition before differentiation induction; and
(ii) a step of evaluating an ability to differentiate into a specific cell using the measured phenotype as an indicator.

FIG. 1

## FIG. 2

NANOG

## FIG. 3

N-CADHERIN

# FIG. 4

VIMENTIN

# FIG. 5

NUCLEAR AREA

## FIG. 6

RATIO OF AVERAGE NUCLEAR AREA/AVERAGE CYTOPLASMIC AREA

## FIG. 7

# FIG. 8

## LACTIC ACID

# FIG. 9

## N-CADHERIN

# FIG. 10

VIMENTIN

# FIG. 11

PAX6

## INTERNATIONAL SEARCH REPORT

| International application No. |
|---|
| PCT/JP2021/001152 |

A. CLASSIFICATION OF SUBJECT MATTER
C12N 5/071(2010.11)i; C12N 5/0735(2010.01)i; C12N 5/077(2010.01)i; C12N 5/10(2006.01)i; C12Q 1/06(2006.01)i
FI: C12N5/071; C12N5/0735; C12N5/077; C12N5/10; C12Q1/06
According to International Patent Classification (IPC) or to both national classification and IPC

B. FIELDS SEARCHED

Minimum documentation searched (classification system followed by classification symbols)
C12N5/071; C12N5/0735; C12N5/077; C12N5/10; C12Q1/06

Documentation searched other than minimum documentation to the extent that such documents are included in the fields searched
| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreaIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | WO 2019/078342 A1 (OSAKA UNIVERSITY) 25 April 2019 (2019-04-25) in particular, example 1 | 12, 15 |
| Y | in particular, claims 1–25, paragraphs [0004]–[0008], examples 1–4 | 1-11, 13, 14, 16 |

☒ Further documents are listed in the continuation of Box C.  ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 26 February 2021 (26.02.2021) | 16 March 2021 (16.03.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**EP 4 092 107 A1**

**INTERNATIONAL SEARCH REPORT**

International application No.

PCT/JP2021/001152

C (Continuation). DOCUMENTS CONSIDERED TO BE RELEVANT

| Category* | Citation of document, with indication, where appropriate, of the relevant passages | Relevant to claim No. |
|---|---|---|
| Y | HOFFDING, M. K. and HYTTEL, P., "Ultrastructural visualization of the Mesenchymal-to-Epithelial Transition during reprogramming of human fibroblasts to induced pluripotent stem cells", Stem Cell Research, 2015, vol. 14, pp. 39-53 in particular, abstract, page 40, left column, paragraph [0002], page 42, left column, paragraph [0003] to page 47, right column, paragraph [0001], page 47, right column, paragraph [0003] to page 48, right column, paragraph [0002], fig. 1, 3, 5, 6 | 1-11, 13, 14, 16 |
| A | in particular, abstract, page 40, left column, paragraph [0002], page 42, left column, paragraph [0003] to page 47, right column, paragraph [0001], page 47, right column, paragraph [0003] to page 48, right column, paragraph [0002], fig. 1, 3, 5, 6 | 12, 15 |
| Y | EASTHAM, A. M. et al., "Epithelial-Mesenchymal Transition Events during Human Embryonic Stem Cell Differentiation", Cancer Research, 2007, vol. 67, no. 23, pp. 11254-11262 in particular, abstract, page 11254, left column, paragraph [0001] to right column, paragraph [0001], fig. 1 | 4-9 |
| A | in particular, abstract, page 11254, left column, paragraph [0001] to right column, paragraph [0001], fig. 1 | 1-3, 10-16 |
| Y | BHOWMIK, S. K. et al., "EMT-induced metabolite signature identifies poor clinical outcome", Oncotarget, 2015, vol. 6, no. 40, pp. 42651-42660 in particular, abstract, fig. 1 | 4-9 |
| A | in particular, abstract, fig. 1 | 1-3, 10-16 |
| A | MALDONADO, M. et al., "ROCK inhibitor primes human induced pluripotent stem cells to selectively differentiate towards mesendodermal lineage via epithelial-mesenchymal transition-like modulation", Stem Cell Research, 2016, vol. 17, pp. 222-227 entire text | 1-16 |
| A | LI, Y. et al., "Reprogramming somatic cells to pluripotency", Cell Cycle, 2013, vol. 12, no. 23, pp. 3594-3598 entire text | 1-16 |
| A | TESHIGAWARA, R. et al., "Visualization of sequential conversion of human intermediately reprogrammed stem cells into iPS cells", Genes to Cells, 2019, vol. 24, pp. 667-673 entire text | 1-16 |

Form PCT/ISA/210 (continuation of second sheet) (January 2015)

**INTERNATIONAL SEARCH REPORT**
Information on patent family members

International application No.

PCT/JP2021/001152

| Patent Documents referred in the Report | Publication Date | Patent Family | Publication Date |
|---|---|---|---|
| WO 2019/078342 A1 | 25 Apr. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010158206 A
- JP 2019106999 A
- JP 2019003336 W
- JP 2019004553 W
- JP 5984217 B

### Non-patent literature cited in the description

- Manipulating the Mouse Embryo. A Laboratory Manual. Cold Spring Harbor Laboratory Press, 1994
- THOMASON, J. A. et al. *Science,* 1998, vol. 282, 1145-1147
- WILMUT et al. *Nature,* 1997, vol. 385, 810
- CIBELLI et al. *Science,* 1998, vol. 280, 1256
- AKIRA IRIYA et al. *Protein, nucleic acid, and enzyme,* 1999, vol. 44, 892
- BAGUISI et al. *Nature Biotechnology,* 1999, vol. 17, 456
- WAKAYAMA et al. *Nature,* 1998, vol. 394, 369
- *Nature Genetics,* 1999, vol. 22, 127
- *Proc. Natl. Acad. Sci. USA,* 1999, vol. 96, 14984
- RIDEOUT III et al. *Nature Genetics,* 2000, vol. 24, 109
- TACHIBANA et al. Human Embryonic Stem Cells Delivered by Somatic Cell Nuclear Transfer. *Cell,* 2013
- KIM et al. *Science,* 2007, vol. 315, 482-486
- NAKAJIMA et al. *Stem Cells,* 2007, vol. 25, 983-985
- KIM et al. *Cell Stem Cell,* 2007, vol. 1, 346-352
- REVAZOVA et al. *Cloning Stem Cells,* 2007, vol. 9, 432-449
- REVAZOVA et al. *Cloning Stem Cells,* 2008, vol. 10, 11-24
- STRELCHENKO N. et al. *Reprod Biomed Online,* 2004, vol. 9, 623-629
- KLIMANSKAYA I. et al. *Nature,* 2006, vol. 444, 481-485
- CHUNG Y. et al. *Cell Stem Cell,* 2008, vol. 2, 113-117
- ZHANG X. et al. *Stem Cells,* 2006, vol. 24, 2669-2676
- WASSARMAN, P. M. et al. *Methods in Energy,* 2003, vol. 365
- MATSUI et al. *Cell,* 1992, vol. 70, 841-847
- SHAMBLOTT et al. *Proc. Natl. Acad. Sci. USA,* 1998, vol. 95 (23), 13726-13731
- TURNPENNY et al. *Stem Cells,* 2003, vol. 21 (5), 598-609
- TAKAHASHI K ; YAMANAKA S. *Cell,* 2006, vol. 126 (4), 663-676
- TAKAHASHI, K et al. *Cell,* 2007, vol. 131 (5), 861-72
- YU J et al. *Science,* 2007, vol. 318 (5858), 1917-1920
- NAKAGAWA M et al. *Nat. Biotechnol.,* 2008, vol. 26 (1), 101-106
- KIM J B et al. *Nature,* 2008, vol. 454 (7204), 646-650
- KIM J B et al. *Cell,* 2009, vol. 136 (3), 411-419
- ZHOU H ; WU S ; JOO JY et al. *Cell Stem Cell,* 2009, vol. 4, 381-384
- KIM D ; KIM CH ; MOON JI et al. *Cell Stem Cell,* 2009, vol. 4, 472-476
- HUANGFU D et al. *Nat. Biotechnol.,* 2008, vol. 26 (7), 795-797
- HUANGFU D et al. *Nat. Biotechnol.,* 2008, vol. 26 (11), 1269-1275
- SILVA J et al. *PLoS. Biol.,* 2008, vol. 6 (10), e253
- STADTFELD M et al. *Science,* 2008, vol. 322 (5903), 945-949
- OKITA K et al. *Science,* 2008, vol. 322 (5903), 949-953
- WOLTJEN K ; MICHAEL IP ; MOHSENI P et al. *Nature,* 2009, vol. 458, 766-770
- KAJI K ; NORRBY K ; PAC A A et al. *Nature,* 2009, vol. 458, 771-775
- YUSA K ; RAD R ; TAKEDA J et al. *Nat Methods,* 2009, vol. 6, 363-369
- YU J ; HU K ; SMUGA-OTTO K ; TIAN S et al. *Science,* 2009, vol. 324, 797-801
- MCNAMARA et al. *Cell Systems,* 2018, vol. 7, 359-370